# EUROPEAN PATENT SPECIFICATION

(11) **EP 2 346 484 B1**
(45) Date of publication and mention of the grant of the patent: **06.11.2019**
(21) Application number: 09741229.0
(22) Date of filing: 20.10.2009
(51) Int. Cl.: A61K 8/42, A61K 8/44, A61K 8/46, A61K 8/73, A61K 8/81, A61K 8/84, A61Q 5/04

(54) **COMPOSITION FOR THE PERMANENT SHAPING OF HUMAN HAIR**
ZUSAMMENSETZUNG ZUR DAUERHAFTEN VERFORMUNG DES MENSCHLICHEN HAARES
COMPOSITION DE MISE EN FORME PERMANENTE DE CHEVEUX HUMAINS

(30) Priority: 20.10.2008 EP 08018290
(43) Date of publication of application: 27.07.2011
(73) Proprietor: Kao Germany GmbH, 64297 Darmstadt (DE)
(72) Inventor: WOOD, Jonathan, 69469 Weinheim (DE); HULLMANN, Alexandra, 64331 Weinheim (DE); SCHNEIDER, Jörg, 64347 Griesheim (DE)
(74) Representative: Grit, Mustafa
(86) International application number: PCT/EP2009/007497
(87) International publication number: WO 2010/046076

(56) References cited:
- EP-A- 0 653 202
- EP-A- 1 166 766
- EP-A- 1 362 573
- EP-A- 1 362 574
- EP-A- 1 475 076
- EP-A- 1 797 861
- WO-A-02/055036
- DE-A1- 10 360 688
- FR-A- 2 785 794

## Description

The present invention concerns a composition and process for the permanent shaping of human hair as laid out in the present claims. The composition and process are both used for the permanent waving of human hair with an excellent waving effect as well as for the straightening of either naturally or chemically curled hair comprising a diamide compound and at least one cationic polymer.

It is generally known that permanent waving is carried out in two steps, the reductive splitting of the cysteine disulfide bonds in the hair by a reducing agent, and the subsequent neutralization by application of an oxidizing agent, whereby the cysteine disulfide bonds are restored.

The reducing agent still most frequently used today is thioglycolic acid, also in form of the salts thereof, in particular its ammonium salt, although numerous other thio compounds have been proposed for this purpose, which, however, mostly did not succeed.

The compositions containing thioglycolates are customarily applied at a pH-value between 7 and 10, in particular 8.5 and 9.5.

Such compositions vary in their waving and/or straightening performance and, therefore, there is still need for further improvement.

The present invention starts from the task of providing a composition for the permanent shaping of human hair with excellent waving and straightening performance. Hair waved or straightened with composition disclosed herein looks and feels natural upon touching by hand. For waved hair it is especially important that the hair has excellent elasticity and bounce.

It has surprisingly been observed that a permanent shaping composition based on at least one reducing agent and further comprising at least one compound comprising 2 amide (diamide) groups and at least one cationic polymer has excellent straightening and waving effects and additionally hair either straightened or waved has improved elasticity, feels natural and softer upon touching. The effects are found to be synergistic.

Accordingly, the first object of the present invention is a composition for permanent shaping hair based on at least one reducing agent and further comprising at least one compound comprising at least two amide (diamide) groups in its molecule and at least one cationic polymer.

Diamide compounds have been known in the art. EP 1362573 A1 discloses compositions comprising a diamide compound and alkalizing agent at a pH between 12 and 14 for hair straightening. The documents is silent on the use of reducing agents.

US 6,685,953 is on external preparations comprising a diamide compound. EP 1366753 A2 is on hair dye compositions comprising diamide compound. Reducing agent, sodium sulphite, is used to stabilize oxidative dye precursors. Nothing is disclosed on permanent shaping of hair.

EP 1362574 A1 discloses permanent shaping compositions based on at least one reducing agent and comprising a diamide compound. The document is silent on cationic polymers.

Use of cationic polymers in permanent shaping compositions has been known from numerous scientific and/or patent publications such as EP 797 861 A1, EP 1797861 A1 and DE 103 60 688 A1. None of them include a hint to combine cationic polymer with a diamide compound.

Permanent shaping compositions comprise at least one diamide compound. Preferred diamide compounds are according to the general structure 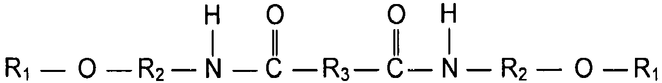 wherein R₁ is a linear or branched, saturated or unsaturated alkyl chain with 1 to 12 C atoms which may be substituted with hydroxy and/or alkoxy groups, preferably R₁ is linear or branched, saturated or unsaturated alkyl chain with 1 to 12 C atoms which may be substituted by 1 to 3 substituents selected from a hydroxy group and C1 to C6 alkoxy group, more preferably R₁ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₂ is linear or branched alkyl chain with 1 to 5 C atoms, preferably linear or branched alkyl chain with 2 to 5 C atoms and more preferably an alkyl chain with 2 to 3 C atoms, and R₃ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the formula A to G.

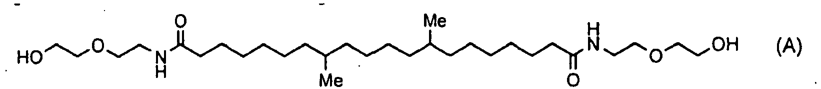

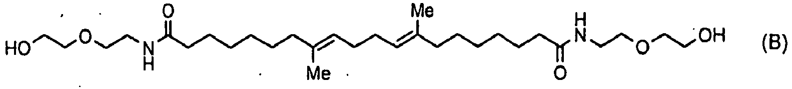

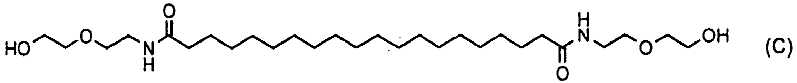

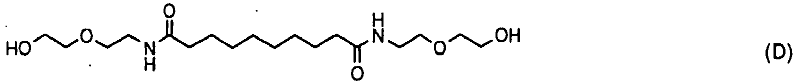

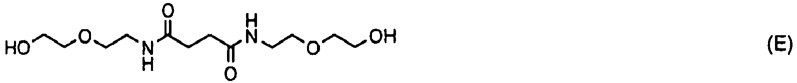

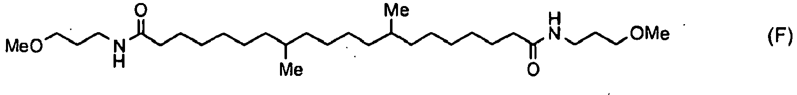

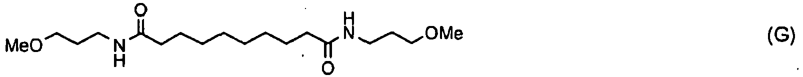

Particularly preferred diamide compound is the compound F which is bis (methoxypropylamido) isodocosane and commercially available from Kao Corporation - Japan.

Concentration of diamide compounds in the compositions of the present invention is in the range of 0.001 to 5%, preferably 0.002 to 3% more preferably 0.005 to 2% and most preferably 0.01 to 1% by weight calculated to total composition.

The permanent shaping compositions according to the invention comprise at least one reducing agent at a concentration of at least 0.5% by weight calculated to total composition. Preferred are thioglycolic acid and thiolactic acid as well as the salts thereof, in particular the ammonium and ethanolamine salts. Further useful thio compounds are in particular cysteine or the hydrochloride thereof, homocysteine, cysteamine, N-acetyl cysteine, thioglycerol, ethanediol monothioglycolate, 1.2-propyleneglycol monothioglycolate (see also WO-A 93/1791), 1-3-propanediol monothioglycolate or the isomer mixture resulting therefrom, 1.3-butanediol and 1.4-butanediol monothioglycolate and the isomer mixtures therefrom, polyethylene glycol, such as di-, tri- and tetraethyleneglycol monothioglycolates, glycerol monothiolactate and further thio acids and the esters thereof, as well as mixtures thereof.

The use of inorganic reducing sulfur compounds such as sodium hydrogen sulfite is basically also possible.

The total reduction agent content in the compositions according to the invention customarily amounts from 0.5 to 15 %, preferably 1.0 to 12.5%, more preferably 1.5 to 12.5%, most preferably 2.0 to 12.5% by weight, calculated to total composition as free thioglycolic acid as reference substance.

Permanent shaping compositions of the present invention comprise at least one cationic polymer. Basically suitable are all cationic polymers listed under the generic name "Polyquaternium" in the CTFA International Cosmetic Ingredient Dictionary. Preferred are Polyquaternium 2, Polyquaternium 4, Polyquaternium 5, Polyquaternium 6, Polyquaternium 7, Polyquaternium 10, Polyquaternium-11, Polyquaternium 16, Polyquaternium 22; Polyquaternium 28, Polyquaternium 39, and Polyquaternium-87.

The cationic polymers also include the quaternized products of graft polymers from organopolysiloxanes and polyethyl oxazolines described in EP-A 524 612 and EP-A 640 643. Such polymer is known with its CTFA name Polysilicone-9.

It should be noted that composition can comprise more than one cationic polymer and especially mixture of quaternized products of graft polymers from organopolysiloxanes and polyethyl oxazolines such as Polysilicone-9 and at least one compound known with the general CTFA name Polyquaternium.

Concentration of total cationic polymer is in the range from of 0.01 to 5%, preferably 0.01 to 3% more preferably 0.02 to 2% and most preferably 0.05 to 1% by weight calculated to total composition.

The permanent shaping compositions containing reducing agents can, if necessary, comprise alkalizing agents. Their quantity is dependent on the reducing agent and the desired pH-value of the composition. Reducing agent compositions preferably contain 0.1% to 5%, in particular 0.5% to 2.5% by weight thereof, calculated to the total composition. Alkalizing agents preferred within the scope of the invention are ammonium carbamate, ammonia and/or ammonium(bi)carbonate, monoethanolamine and triethanolamine. It is desirable to adjust the pH-value between about 6.5 and 10.5, preferably about 7 to 9.5.

The permanent shaping compositions according to the invention are suited for use both for the permanent waving, i.e. curling of human hair and for the straightening, i.e. smoothing thereof.

The viscosity best suited for the permanent shaping compositions according to the invention proved to be in the range of 1 to 10,000 mPa.s, preferably about 1 to about 5,000 mPa.s, measured at 20°C in a Brookfield viscosimeter (no. 5 spindle), whereas the viscosity suited for the straightening compositions is preferably higher in a range up to 50,000 mPa.s, preferably up to 30,000 mPa.s measured at 20°C in a Brookfield viscosimeter (no. 5 spindle).

The viscosity is adjusted by addition of the appropriate amounts of thickening agents known per se, such as cellulose derivatives. Thickening may as well be realized by formulating a composition in form of an emulsion with the use of C₁₀-C₂₂-fatty alcohols, in mixture with long mono alkyl chain quaternary ammonium surfactants.

The permanent shaping compositions according to the present invention preferably comprise surfactants selected from anionic, nonionic, cationic and amphoteric ones. Their proportion ranges from 0.05 % to 10%, in particular from 0.1 % to 5 % by weight, calculated to total composition.

Suitable anionic surfactants are especially the known alkyl ether sulfates and carboxylic acids, in particular in form of their alkali salts, as well as protein fatty acid condensates.

Suitable nonionic surfactants, which are preferred within the scope of the invention, are in particular C₈-C₁₈-fatty alcohol polyglycol ethers, fatty acid polyglycol esters, fatty acid alkanolamides, amineoxides, and especially C₈-C₁₈-alkyl polyglucosides.

Also possible is the incorporation of amphoteric surfactants, such as the known alkyl betaines, alkyl amido betaines, and alkyl amphoacetates.

Further according to a further preferred embodiment, permanent shaping compositions comprise at least one cationic surfactant according to general formula 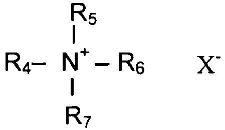
where R₄ is a saturated or unsaturated, branched or non-branched alkyl chain with 8-24 C atoms or

   R₈CO NH (CH₂)ₙ

   where R₈ is saturated or unsaturated, branched or non-branched alkyl chain with 7-21 C atoms and n has typical value of 0 - 4 or

   R₉CO O (CH₂)ₙ

   where R₉ is saturated or unsaturated, branched or non-branched alkyl chain with 7-21 C atoms and n has typical value of 0 - 4, and
R₅ is a hydrogen, saturated or unsaturated, branched or non-branched alkyl chain with 1-24 C atoms or

   R₈CO NH (CH₂)ₙ

   where R₈ is saturated or unsaturated, branched or non-branched alkyl chain with 7-21 C atoms and n has typical value of 0 - 4 or

   R₉ CO O (CH₂)ₙ

   where R₉ is saturated or unsaturated, branched or non-branched alkyl chain with 7-21 C atoms and n has typical value of 0 - 4,
and R₆ and R₇ are independent from each other H or lower alkyl chain with 1 to 4 carbon atoms which may be substituted with one or more hydroxyl group, and X is chloride, bromide or methosulfate.

Concentration of cationic surfactant is in the range from 0.05 % to 5 %, preferably 0.1% to 2.5 % by weight, calculated to total composition.

Suitable long-chain quaternary ammonium compounds are in particular cetyl trimethyl ammonium chloride, dimethyl dicetyl ammonium chloride, trimethyl cetyl ammonium bromide chloride, stearyl trimethyl ammonium chloride, dimethyl stearyl benzyl ammonium chloride, benzyl tetradecyl dimethyl ammonium chloride, dimethyl dihydrogenated tallow ammonium chloride, lauryl pyridinium chloride, lauryl dimethyl benzyl ammonium chloride, lauryl trimethyl ammonium chloride, tris-(oligooxyethyl) alkyl ammonium phosphate, cetyl pyridinium chloride, etc.

One or more aminated silicones may be used in the compositions of the present invention. Suitable ones are any silicone compound having at least one amine group in its molecule. Well know and widely used example is amodimethicone available as an emulsion from various suppliers either as a cationic emulsion or as a non-ionic emulsion. Advantageously both types are suitable. Concentration varies in the range of 0.05 to 2.5% by weight calculated to total composition.

Any non-ionic surfactant is in principal suitable as emulsifier in aminated silicone emulsion. Suitable ones are those of alkyl polyglucosides and especially decyl or lauryl polyglucosides known with the trade name Plantacare from Cognis and those of ethoxylated fatty alcohol with number of ethoxylation degree between 10 to 50 and prefereably around 20 to 30. Examples to preferred ethoxylated fatty alcohols are Laureth-20, Steareth-20, Myreth-20, Ceteareth-20, Laureth-30, Steareth-30, Myreth-30, Ceteareth-30, etc.

Among the non-ionic surfactants the most preferred non-ionic surfactants are the ones according to formula

CH₂ (CH₂)₁₂ (OCH₂CH₂)ₙ OH

wherein n is a number between 2 and 20. Preferred ones have an n between 4 and 10. Such are known with the CTFA name Trideceth and with a number defining the number of ethoxy groups.

In the most preferred embodiment of the present invention, suitable aminated silicone is amodimethicone and emulsified with Trideceth-5 and Trideceth-10. Such raw material is commercially available under the trade name Belsil ADM 8020 VP from Wacker Chemie.

Permanent shaping compositions of present invention can comprise additionally at least one organic solvent. Suitable organic solvents are 2-methyl-1,3-propanediol, mono and dialcohols or the ethers thereof, in particular mono-C₁-C₃-alkyl ether, ethanol, n-propanol, isopropyl alcohol, 1-methoxypropanol, 1-ethoxypropanol and ethoxydiglycol, diols and their esters 1,3- and 1,4-butanediol, diethyleneglycol and the monomethyl and monoethyl ether thereof, dipropylene glycol and the monomethyl and monoethyl ether thereof, glycerol, hexanetriol, ethyl carbitol, benzyl alcohol, benzyloxy ethanol, propylene carbonate, N-alkyl pyrrolidone, and urea or their mixture preferably in an amount from about 0.1 % to 10 % by weight, calculated to the total composition.

Permanent shaping composition of the present invention can comprise further ceramide type of compound such as cetyl-PG-hydroxyethylpalmitamide.

Further optional ingredient are sterols,especially the phytosterols as preferred hair restructuring agents. Especially preferred ones are of plant origin for example ergosterol, sitosterol, stigmasterol, fucosterol, brassicasterol, fungisterol, campesterol, zymosterol, ascosterol, cerevisterol, episterol, faecosterol, spinasterol. Among those phytosterols, the ones found in "Avocadin" which is the unsaponified fraction of the avocado oil is more preferred.

Optionally fatty acids of C₁₀ to C₂₂ may be incorporated into the compositions of the present invention at a concentration of preferably 0.01 to 2.5% by weight calculated to total composition.

Additionally, one or more natural oil component may be incorporated into the compositions of the present invention. Suitable are such as olive oil, almond oil, avocado oil, wheatgerm oil, ricinus oil or their mixture. Concentration of these natural oil ingredients should be 0.01 to 2.5%, preferably 0.01.to 1%, more preferably 0.05 to 0.5% by weight, calculated to total composition.

Further additional compounds may be present in the permanent shaping compositions of the present invention is ubichinone of the formula 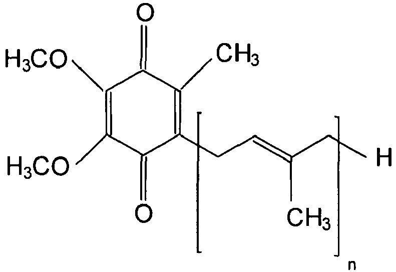 where n is a number between 1 and 10. Preferred ubichinones are the ones where n is a number between 6 and 10 and especially preferred is Ubichinone 50 where n is 10, also known as Coenzyme Q10. Concentration ubichinone of the above formula in permanent shaping compositions of the present invention is from 0.0001 to 1%, preferably from 0.0002 to 0.75%, more preferably from 0.0002 to 0.5% and most preferably from 0.0005 to 0.5% by weight, calculated to total composition.

The compositions used according to the invention can naturally comprise all the substances customarily found in permanent shaping compositions, a list of which will not be given here, and are preferably present as solutions, gels with a higher or lower viscosity, emulsions or creams. They can be single-phase products or compositions packed into separate packaging which are united upon application, as they are disclosed, for example, in DE-C 43 04 828.

In order to avoid repetition, reference is here made to the state of the art as it is described, for example, in "Ullmann's Encyclopedia of Industrial Chemistry", Vol. A12 (1986), pages 588 to 591, and in particular to the monography of K. Schrader, "Grundlagen und Rezepturen der Kosmetika", 2nd. Ed. (1989, Hüthig Buchverlag) pages 823 to 840, as well as the article by D. Hollenberg et. al. in "Seifen-Öle-Fette-Wachse", 117 (1991), pages 81 to 87.

Composition of the present invention is used in a process for permanent waving wherein hair is washed or shampooed first and wound on the curlers, subsequently a reducing composition comprising at least one reducing agent, at least one diamide compound and at least one cationic polymer is applied onto hair and after 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair. In cases where additional conditioning composition is required this may as well be applied after finishing the process as described above.

In another process as described above the curlers are removed after rinsing off the reducing agent and before applying the oxidizing agent.

Further in another process, after rinsing off the reducing agent from hair, an intermediate treatment composition is applied onto hair and without rinsing off, but after removing the excess amount of intermediate treatment with a towel, oxidizing composition is applied and at the end of the processing they are rinsed off from hair and curlers are removed from hair.

It has further been found out that the use of diamide compound and cationic polymer in the intermediate treatment composition improves the permanent shaping of hair as well in terms of curl appearance and natural look and feel of hair. Therefore, in another preferred form of the present invention, permanent shaping of hair is carried out with a process wherein hair is washed or shampooed first and subsequently a composition comprising reducing agent, at least one diamide compound and at least one cationic polymer is applied onto hair and after 1 to 45 min, preferably 1 to 30 min of processing time, depending on the hair strength, rinsed off from hair with tap water and an intermediate treatment composition comprising at least one diamide compound and at least one cationic polymer is applied and without rinsing off an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off. In cases where additional conditioning composition is required this may as well be applied after finishing the above process. In case of permanent waving hair in the above process curlers are put onto hair before application of reducing composition and taken off from hair before application of oxidizing composition or after application and processing of the oxidizing composition.

The intermediate treatment composition has a pH value between 2.5 to 6, preferably 3 to 5.5 and most preferably 3 to 5.

A straightening process may also be carried out in a different process wherein hair is washed and/or shampooed and dried and reducing composition comprising at least one reducing compound and at least one diamide compound and at least one cationic polymer is applied onto dry hair and processed for 5 to 60 min, preferably 5 to 45 min and rinsed off with water and dried and the dry hair physically straighten with hot iron at a temperature of 130 to 210°C and subsequently an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off.

Present invention is also on a kit for permanent shaping hair which comprises a first product containing a composition comprising at least one reducing compound, at least one diamide compound and at least one cationic polymer and a second product containing a composition comprising at least one oxidizing agent and optionally a third product comprising at least one diamide compound and at least one cationic polymer.

The following examples are to illustrate the invention, but not to limit it.

### Example 1:

**Alkaline Permanent Wave for Normal Hair**

| | |
|---|---|
| Ammonium thioglycolate (60%) | 21.3 (% by wt.) |
| Ammonium hydrogen carbonate | 5.0 |
| 1,3- butylene gylcol | 3.0 |
| Diamide compound* | 0.02 |
| Polyquaternium-6 | 0.2 |
| PEG-40-Hydrogenated castor oil | 0.7 |
| Perfume | 0.4 |
| Ammonia, 25% | ad pH 8.3 |
| Water | ad 100.0 |

| | |
|---|---|
| * Bis (methoxypropylamido) isodocosane which corresponds to compound F among the structures disclosed above. | |

With this composition the hair was permanently waved. First the above composition was applied and processed for about 15 minutes, rinsed and neutralized for about 8 minutes with a customary 2.5% H₂O₂ composition. Homogeneous wave appearance was obtained. Exclusion of diamide compound resulted in less homogeneous perm appearance.

Two comparative compositions were prepared comprising either diamide compound or cationic polymer at concentration twice of the above compositions in order to keep the total concentration constant. Hair was waved according to the above described process. It was observed that the composition according to the invention resulted in far better waves which were homogeneous and had improved elasticity and especially hair felt softer upon touching.

### Example 2:

**Alkaline Permanent Wave for Normal Hair**

| | |
|---|---|
| Ammonium thioglycolate (60%) | 20 (% by wt.) |
| Ammonium hydrogen carbonate | 4.0 |
| 1,3- butylene gylcol | 3.0 |
| Amodimethicone* | 0.25 |
| Diamide compound** | 0.05 |
| Polyquaternium-16 | 0.3 |
| PEG-40-Hydrogenated castor oil | 0.7 |
| Perfume | 0.4 |
| Ammonia, 25% | ad pH 8.3 |
| Water | ad 100.0 |

| | |
|---|---|
| * Used as the raw material Belsil ADM 8020 VP The number in the above formula refers to active amodimethicone **Bis (methoxypropylamido) isodocosane which corresponds to compound F among the structures disclosed above. | |

With this composition the hair was permanently waved. First the above composition was applied and processed for about 15 minutes, rinsed and neutralized for about 8 minutes with a customary 2.5% H₂0₂ composition. Homogeneous wave appearance was obtained. Exclusion of diamide compound and polyqauternium-16 resulted in less homogeneous perm appearance.

### Example 3:

**Alkaline Permanent Wave for Damaged Hair**

| | |
|---|---|
| Ammonium thioglycolate (60%) | 15.0 (% by wt.) |
| Ammonium hydrogen carbonate | 2.5 |
| Ceteth-20 | 0.7 |
| Cetrimonium chloride | 0.5 |
| 1,3- butylene gylcol | 0.5 |
| Diamide compound | 0.04 |
| Polyquaternium-6 | 0.8 |
| Perfume | 0.4 |
| Ammonia, 25% | ad pH 8.0 |
| Water | ad 100.0 |

| | |
|---|---|
| * Bis (methoxypropylamido) isodocosane which corresponds to compound F among the structures disclosed above. | |

The permanent wave achieved with this composition was similar to the one obtained with the composition according to Example 1.

Exclusion of the diamide compound and polyquaternium - 6 led to waves with substantially weaker contours.

### Example 4:

**Alkaline Permanent Wave for Damaged Hair**

| | |
|---|---|
| Ammonium thioglycolate (60%) | 0.9 (% by wt.) |
| Cystein hydrochloride | 5.7 |
| Ammonium hydrogen carbonate | 1.5 |
| Acetylcystein | 0.7 |
| Cetrimonium chloride | 0.5 |
| 1,3- butylene gylcol | 0.5 |
| Diamide compound* | 0.03 |
| Polysilicone-9 | 0.3 |
| Coenzyme Q10 | 0.05 |
| Oleic acid | 0.05 |
| Perfume | 0.4 |
| Ammonia, 25% | ad pH 9.8 |
| Water | ad 100.0 |

| | |
|---|---|
| * Bis (methoxypropylamido) isodocosane which corresponds to compound F among the structures disclosed above. | |

The permanent wave achieved with this composition was similar to the one obtained with the composition according to Example 1.

Exclusion of the diamide compound and polysilicone-9 led to substantially weaker waves.

### Example 5

### Neutral Permanent Wave for Normal Hair

A permanent waving product consisting of two Compositions A and B, filled into a two-chamber packaging the chambers of which were kept separate until application, was prepared by destruction of the separating wall and applied onto human hair rolled onto curlers. The hair was rinsed after about fifteen minutes processing and neutralized for about five minutes with a 2.5 % H₂O₂ neutralizer composition, rinsed again, shampooed and dried.

An expressive, even, intensive permanent wave was obtained.

An identical treatment which had no diamide compound and Polyquaternium-87 showed a visibly inferior wave.

**Composition A:**

| | |
|---|---|
| Ammonium hydrogen carbonate | 4.5 (g) |
| Diamide compound* | 0.05 |
| Polyquaternium-87 | 0.5 |
| PEG-65-Hydrogenated castor oil | 0.8 |
| Isopropyl alcohol | 1.5 |
| Ethoxydiglycol | 2.0 |
| Cocoamidopropyl betaine | 1.0 |
| Perfume | 0.3 |
| Coenzyme Q10 | 0.05 |
| Turbidifying agent | 0.5 |
| Ammonia, 25% | ad pH 8.4 |
| Water | ad 72.0 |

| | |
|---|---|
| * Bis (methoxypropylamido) isodocosane which corresponds to compound F among the structures disclosed above. | |

**Composition B:**

| | |
|---|---|
| Ammonium thioglycolate, 70% | 18.0 (g) |
| Thiolactic acid | 2.0 |
| 2-Methyl-1.3-propanediol | 0.5 |
| Ammonia, 25% | ad pH 5.5 |
| Water | ad 28.0 |

After mixing of both Compositions a ready-to-use product with a pH-value of 7.4 was obtained.

### Example 6:

### Neutral Permanent Wave for Dyed Hair

A permanent waving product filled into a two-chamber package was prepared in analogy to Example 4:

**Composition A:**

| | |
|---|---|
| Ammonium hydrogen carbonate | 3.5 (g) |
| Diamide compound * | 0.5 |
| Polysilicone-9 | 0.5 |
| Ethanol | 0.5 |
| 1-Methoxypropanol | 1.0 |
| Cocoamidopropyl betaine | 1.0 |
| PEG-25-glyceryl cocoate | 0.8 |
| Coenzyme Q10 | 0.1 |
| Oleic acid | 0.05 |
| Perfume | 0.3 |
| Turbidifying agent | 0.5 |
| Ammonia, 25% | ad pH 8.3 |
| Water | ad 72.0 |

| | |
|---|---|
| * Bis (methoxypropylamido) isodocosane which corresponds to compound F among the structures disclosed above. | |

**Composition B:**

| | |
|---|---|
| Ammonium thioglycolate, 70% | 13.0 (g) |
| Thiolactic acid | 0.5 |
| 2-Methyl-1.3-propanediol | 1.5 |
| Ammonia, 25% | ad pH 5.5 |
| Water | ad 28.0 |

A product with a pH-value of 7.4 was obtained by mixing of the Compositions immediately prior to application. After application onto dyed hair as described in Example 3, this mixture resulted in an expressive permanent wave, which had not effect whatever on the color gloss and color intensity.

### Example 7:

**Alkaline Permanent Waving Gel**

| | |
|---|---|
| Ammonium thioglycolate, 70% | 15.0 (g) |
| Ammonium hydrogen carbonate | 4.5 |
| PEG-40-Hydrogenated castor oil | 0.7 |
| C₁₂-C₁₈-Fatty alcohol mixture | 3.5 |
| Cetrimonium chloride | 2.0 |
| Diamine compound* | 0.1 |
| 2-Methyl-1.3-propanediol | 0.5 |
| Polyquaternium -6 | 0.4 |
| Coenzyme Q10 | 0.1 |
| Perfume | 0.3 |
| Ammonia, 25% | ad pH 8.0 |
| Water | ad 100.0 |

**Intermediate treatment composition**

| | |
|---|---|
| Asparagic acid | 0.25% by weight |
| Glutamic acid | 0.50 |
| Alanin DL | 0.25 |
| Magnesium sulfate | 10.00 |
| Diamine compound* | 0.01 |
| PEG-40 Hydrogenated castor oil | 0.5 |
| Poyquaternium-16 | 0.2 |
| Water | q.s. to 100 |

| | |
|---|---|
| * Bis (methoxypropylamido) isodocosane which corresponds to compound F among the structures disclosed above. | |

The above composition had a pH of 4.10.

### Example 8:

**Straightening Composition**

| | |
|---|---|
| Thioglycolic acid | 8.0 (% by wt.) |
| C₁₆-C₂₂-Fatty alcohol mixture | 3.5 |
| Oleth-50 | 2.5 |
| Laureth-23 | 1.5 |
| Diamide compound* | 0.3 |
| Polyquaternium-6 | 0.5 |
| Ethanol | 5.0 |
| Perfume | 0.6 |
| Monoethanolamine | ad pH 9.3 |
| Water | ad 100.0 |

| | |
|---|---|
| * Bis (methoxypropylamido) isodocosane which corresponds to compound F among the structures disclosed above. | |

This composition constitutes an effecting smoothing composition for kinky hair.

## Claims

1. A composition for the permanent shaping of human hair **characterized in that** it comprises at least one reducing agent, at least one compound with at least two amide groups (diamide compound) in its molecule is selected from compounds according to general structure 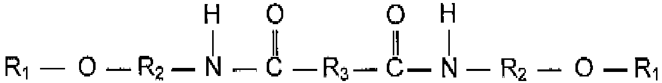 wherein R₁ is a linear or branched, saturated or unsaturated alkyl chain with 1 to 12 C atoms which may be substituted with hydroxy and/or alkoxy groups, R₂ is linear or branched alkyl chain with 1 to 5 C atoms, and R₃ linear or branched, saturated or unsaturated alkyl chain with 1 to 22 C atoms.and at least one cationic polymer selected from Polyquaternium 2, Polyquaternium 4, Polyquaternium 5, Polyquaternium 6, Polyquaternium 7, Polyquaternium 10, Polyquaternium-11, Polyquaternium 16, Polyquaternium 22, Polyquaternium 28, and Polyquaternium 39, Polyquaternium-87 and quaternized products of graft polymers from organopolysiloxanes and polyethyl oxazolines.

2. The composition according to claim 1 **characterized in that** the reducing agent is selected from thioglycolic acid, thiolactic acid and their salts, cystein and its hydrochloride salt, acetylcystein.

3. The composition according to claims 1 and 2 **characterized in that** at least one diamide compound is selected from the compounds

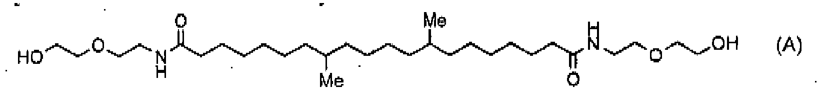

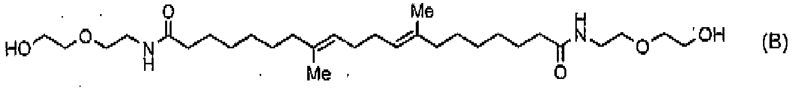

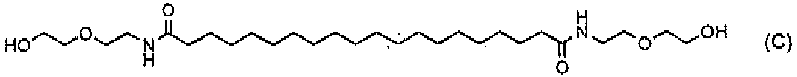

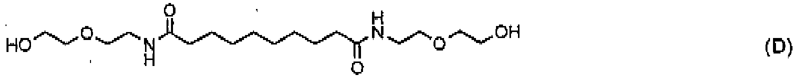

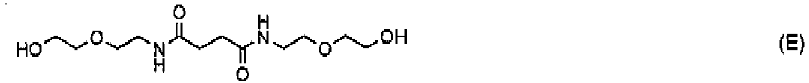

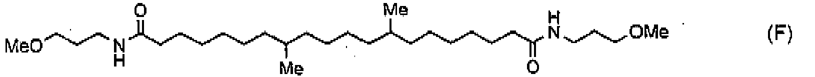

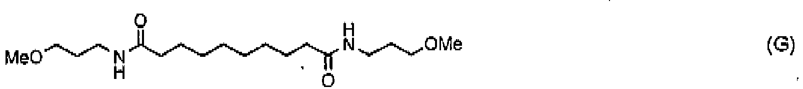

4. The composition according to claim 3 **characterized in that** at least one diamide compound is

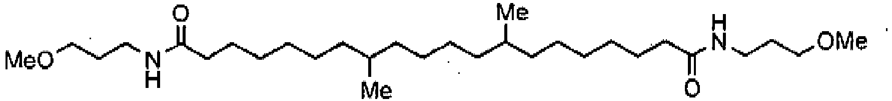

5. The composition according to any of the preceding claims **characterized in that** it comprises reducing agent at a concentration of 0.5 to 15% by weight, diamide compound at a concentration of 0.001 to 5% by weight, and cationic polymer at a concentration of 0,01 to 5% by weight, calculated to total composition.

6. The composition according to any of the preceding claims **characterized in that** it has a pH in the range of 6.5 to 10.5.

7. Process for permanent waving hair **characterized in that** hair is washed or shampooed first and wound on curlers and subsequently a composition comprising at least one reducing agent, at least one compound with at least two amide groups (diamide compound) in its molecule is selected from compounds according to general structure 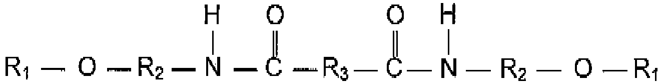 wherein R₁ is a linear or branched, saturated or unsaturated alkyl chain with 1 to 12 C atoms which may be substituted with hydroxy and/or alkoxy groups, R₂ is linear or branched alkyl chain with 1 to 5 C atoms, and R₃ linear or branched, saturated or unsaturated alkyl chain with 1 to 22 C atoms.and at least one cationic polymer is applied onto hair and at the end of the 1 to 45 min, preferably 1 to 30 min of processing time, depending on the hair strength, rinsed off from hair with tap water and an oxidizing composition comprising at least one oxidizing agent, preferably hydrogen peroxide or sodium bromate at a concentration of 0.5 to 10% by weight calculated to total of oxidizing composition, is applied onto hair and left on the hair 1 to 20 min and rinsed off and curlers are removed from hair.

8. The process according to claim 7 **characterized in that** the reducing agent is selected from thioglycolic acid, thiolactic acid and their salts, cystein and its hydrochloride salt, acetylcystein.

9. The process according to claims 7 and 8 **characterized in that** at least one diamide compound is selected from the compounds

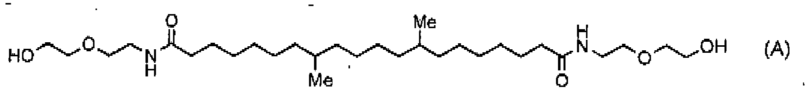

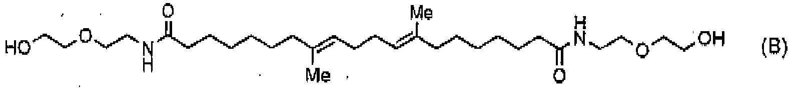

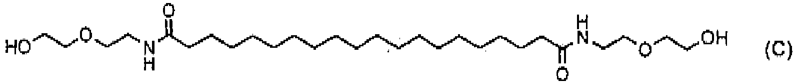

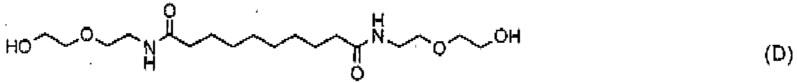

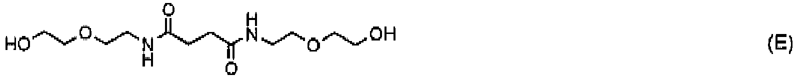

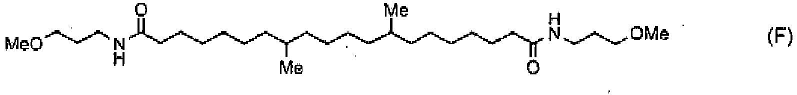

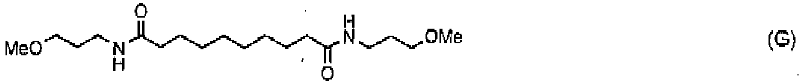

10. The process according to claim 9 **characterized in that** at least one diamide compound is

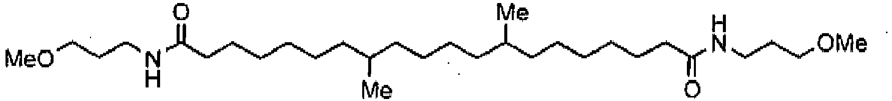

11. The process according to claims 7 to 10 **characterized in that** the composition comprises reducing agent at a concentration of 0.5 to 15% by weight, diamide compound at a concentration of 0.001 to 5% by weight, and cationic polymer at a concentration of 0.01 to 5% by weight, calculated to total composition.

12. The process according to claims 7 to 11 **characterized in that** the composition comprises at least one surfactant selected from anionic, nonionic, cationic and amphoteric ones at a concentration of 0.05 to 10% by weight, calculated to total composition.

13. The process according to claims 7 to 12 **characterized in that** the composition comprises at least one organic solvent at a concentration of 0.1 to 10% by weight, calculated to total composition.

14. The process according to claims 7 to 13 **characterized in that** the composition comprises at least one ubichinone of the formula 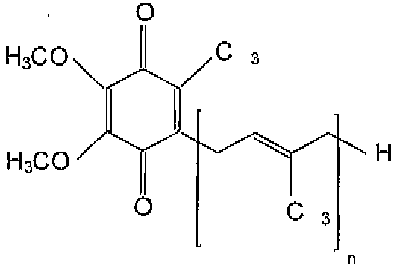 where n is a number between 1 and 10.

15. The process according to claims 7 to 14 **characterized in that** the composition has a pH in the range of 6.5 to 10.5.

## Patentansprüche

1. Zusammensetzung zum dauerhaften Formen von menschlichen Haaren, **dadurch gekennzeichnet, dass** sie mindestens ein Reduktionsmittel, mindestens eine Verbindung mit mindestens zwei Amidgruppen (Diamidverbindung) in ihrem Molekül, ausgewählt aus Verbindungen der allgemeinen Struktur 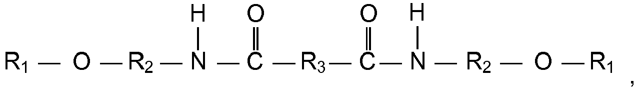 wobei R₁ für eine lineare oder verzweigte, gesättigte oder ungesättigte Alkylkette mit 1 bis 12 C-Atomen steht, welche mit Hydroxy- und/oder Alkoxygruppen substituiert sein kann, R₂ für eine lineare oder verzweigte Alkylkette mit 1 bis 5 C-Atomen steht und R₃ für eine lineare oder verzweigte, gesättigte oder ungesättigte Alkylkette mit 1 bis 22 C-Atomen steht, und mindestens ein kationisches Polymer aufweist, ausgewählt aus Polyquaternium 2, Polyquaternium 4, Polyquaternium 5, Polyquaternium 6, Polyquaternium 7, Polyquaternium 10, Polyquaternium 11, Polyquaternium 16, Polyquaternium 22, Polyquaternium 28 und Polyquaternium 39, Polyquaternium 87 und quaternisierten Produkten von Pfropfpolymeren aus Organopolysiloxanen und Polyethyloxazolinen.

2. Zusammensetzung nach Anspruch 1, **dadurch gekennzeichnet, dass** das Reduktionsmittel aus Thioglykolsäure, Thiomilchsäure und deren Salzen, Cystein und dessen Hydrochloridsalz, Acetylcystein ausgewählt ist.

3. Zusammensetzung nach Anspruch 1 und 2, **dadurch gekennzeichnet, dass** mindestens eine Diamidverbindung aus den Verbindungen 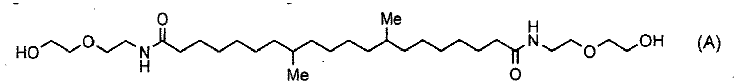 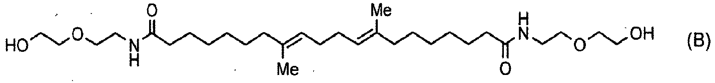 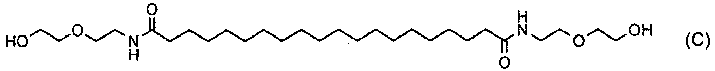 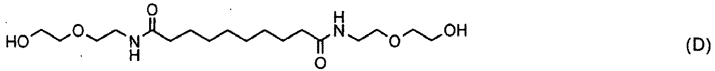 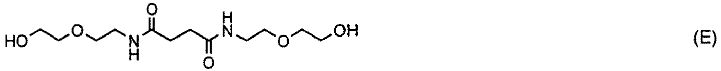 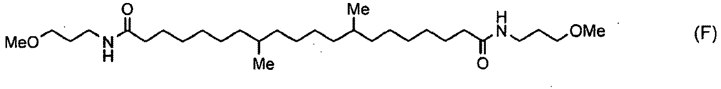 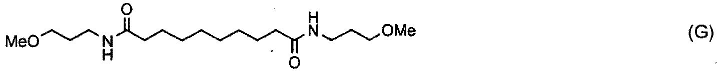 ausgewählt ist.

4. Zusammensetzung nach Anspruch 3, **dadurch gekennzeichnet, dass** mindestens eine Diamidverbindung 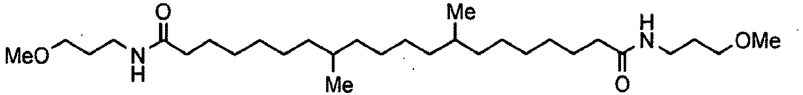 ist.

5. Zusammensetzung nach einem der vorhergehenden Ansprüche, **dadurch gekennzeichnet, dass** sie Reduktionsmittel in einer Konzentration von 0,5 Gewichts-% bis 15 Gewichts-%, Diamidverbindung in einer Konzentration von 0,001 Gewichts-% bis 5 Gewichts-% und kationisches Polymer in einer Konzentration von 0,01 Gewichts-% bis 5 Gewichts-% aufweist, bezogen auf die Gesamtzusammensetzung.

6. Zusammensetzung nach einem der vorhergehenden Ansprüche, **dadurch gekennzeichnet, dass** sie einen pH-Wert im Bereich von 6,5 bis 10,5 aufweist.

7. Verfahren zum dauerhaften Wellen von Haaren, **dadurch gekennzeichnet, dass** die Haare zunächst gewaschen oder shampooniert und auf Lockenwickler gezogen werden und anschließend eine Zusammensetzung, welche mindestens ein Reduktionsmittel, mindestens eine Verbindung mit mindestens zwei Amidgruppen (Diamidverbindung) in ihrem Molekül, ausgewählt aus Verbindungen der allgemeinen Struktur 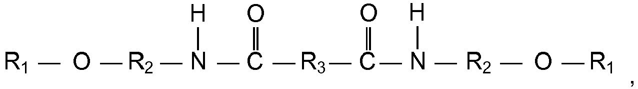 wobei R₁ für eine lineare oder verzweigte, gesättigte oder ungesättigte Alkylkette mit 1 bis 12 C-Atomen steht, welche mit Hydroxy- und/oder Alkoxygruppen substituiert sein kann, R₂ für eine lineare oder verzweigte Alkylkette mit 1 bis 5 C-Atomen steht und R₃ für eine lineare oder verzweigte, gesättigte oder ungesättigte Alkylkette mit 1 bis 22 C-Atomen steht, und mindestens ein kationisches Polymer aufweist, auf die Haare aufgebracht wird und am Ende der 1- bis 45-minütigen, vorzugsweise 1- bis 30-minütigen Einwirkzeit, abhängig von der Stärke der Haare, mit Leitungswasser aus den Haaren ausgespült wird und eine oxidierende Zusammensetzung, aufweisend mindestens ein Oxidationsmittel, vorzugsweise Wasserstoffperoxid oder Natriumbromat in einer Konzentration von 0,5 Gewichts-% bis 10 Gewichts-%, bezogen auf die gesamte oxidierende Zusammensetzung, auf die Haare aufgebracht wird und 1 min bis 20 min auf den Haaren belassen wird und ausgespült wird und die Lockenwickler aus den Haaren entfernt werden.

8. Verfahren nach Anspruch 7, **dadurch gekennzeichnet, dass** das Reduktionsmittel aus Thioglykolsäure, Thiomilchsäure und deren Salzen, Cystein und dessen Hydrochloridsalz, Acetylcystein ausgewählt ist.

9. Verfahren nach Anspruch 7 und 8, **dadurch gekennzeichnet, dass** mindestens eine Diamidverbindung aus den Verbindungen 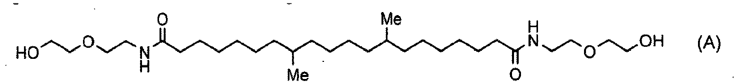 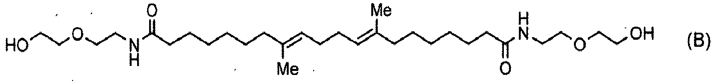 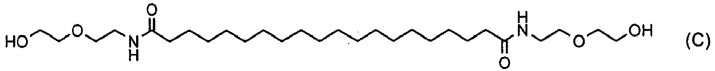 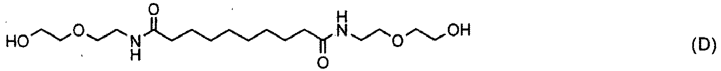 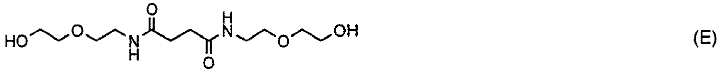 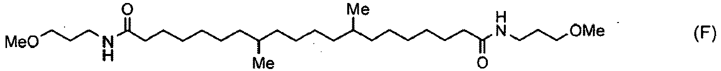 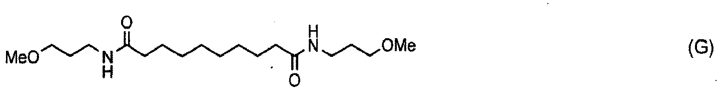 ausgewählt ist.

10. Verfahren nach Anspruch 9, **dadurch gekennzeichnet, dass** mindestens eine Diamidverbindung 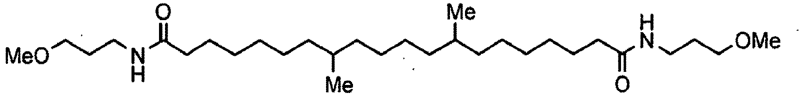 ist.

11. Verfahren nach Anspruch 7 bis 10, **dadurch gekennzeichnet, dass** die Zusammensetzung Reduktionsmittel in einer Konzentration von 0,5 Gewichts-% bis 15 Gewichts-%, Diamidverbindung in einer Konzentration von 0,001 Gewichts-% bis 5 Gewichts-% und kationisches Polymer in einer Konzentration von 0,01 Gewichts-% bis 5 Gewichts-% aufweist, bezogen auf die Gesamtzusammensetzung.

12. Verfahren nach Anspruch 7 bis 11, **dadurch gekennzeichnet, dass** die Zusammensetzung mindestens ein Tensid, ausgewählt aus anionischen, nichtionischen, kationischen und amphoteren, in einer Konzentration von 0,05 Gewichts-% bis 10 Gewichts-% aufweist, bezogen auf die Gesamtzusammensetzung.

13. Verfahren nach Anspruch 7 bis 12, **dadurch gekennzeichnet, dass** die Zusammensetzung mindestens ein organisches Lösungsmittel in einer Konzentration von 0,1 Gewichts-% bis 10 Gewichts-% aufweist, bezogen auf die Gesamtzusammensetzung.

14. Verfahren nach Anspruch 7 bis 13, **dadurch gekennzeichnet, dass** die Zusammensetzung mindestens ein Ubichinon der Formel 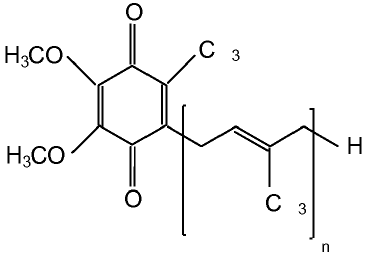 aufweist, wobei n eine Zahl von 1 bis 10 ist.

15. Verfahren nach Anspruch 7 bis 14, **dadurch gekennzeichnet, dass** die Zusammensetzung einen pH-Wert im Bereich von 6,5 bis 10,5 aufweist.

## Revendications

1. Composition pour la mise en forme permanente de cheveux humains **caractérisée en ce qu'**elle comprend au moins un agent de réduction, au moins un composé avec au moins deux groupes amides (composé diamide) dans sa molécule, qui est choisi parmi des composés selon la structure générale 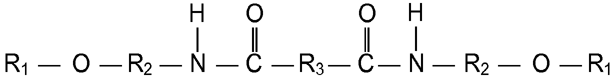 où R₁ est une chaîne alkyle linéaire ou ramifiée, saturée ou non saturée, avec de 1 à 12 atomes C qui peuvent être substitués avec des groupes hydroxyle et/ou alcoxy, R₂ est une chaîne alkyle linéaire ou ramifiée avec de 1 à 5 atomes C, et R₃ est une chaîne alkyle linéaire ou ramifiée, saturée ou non saturée avec de 1 à 22 atomes C et au moins un polymère cationique choisi parmi le Polyquaternium 2, le Polyquaternium 4, le Polyquaternium 5, le Polyquaternium 6, le Polyquaternium 7, le Polyquaternium 10, le Polyquaternium 11, le Polyquaternium 16, le Polyquaternium 22, le Polyquaternium 28 et le Polyquaternium 39, le Polyquaternium 87 et des produits quaternisés à base de polymères greffés à partir d'organopolysiloxanes et de polyéthyl oxazolines.

2. Composition selon la revendication 1, **caractérisée en ce que** l'agent de réduction est choisi parmi l'acide thioglycolique, l'acide thiolactique et leurs sels, la cystéine et son sel chlorhydrate, l'acétylcystéine.

3. Composition selon les revendications 1 et 2, **caractérisée en ce qu'**au moins un composé diamide est choisi parmi les composés

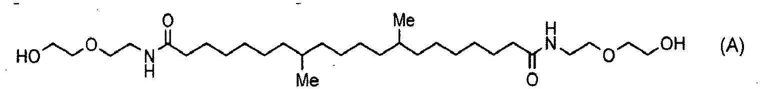

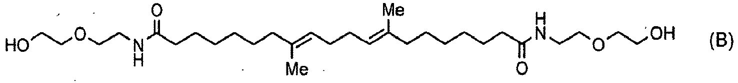

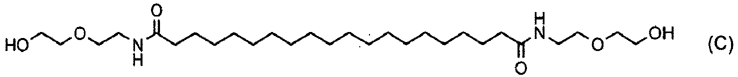

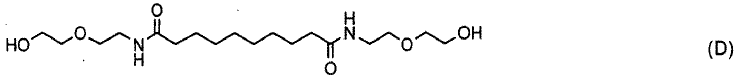

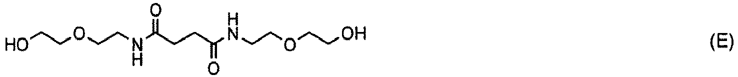

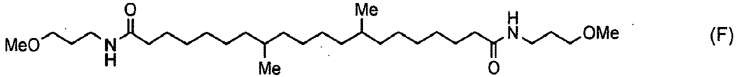

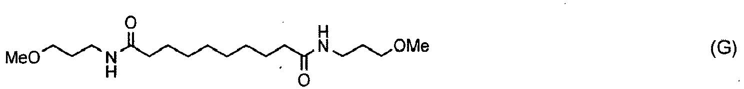

4. Composition selon la revendication 3, **caractérisée en ce qu'**au moins un composé diamide est le

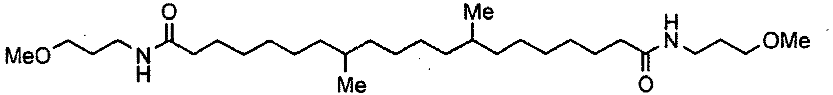

5. Composition selon l'une quelconque des revendications précédentes, **caractérisée en ce qu'**elle comprend un agent de réduction à une concentration de 0,5 à 15 % en poids, un composé diamide à une concentration de 0,001 à 5 % en poids, et un polymère cationique à une concentration de 0,01 à 5 % en poids, calculées par rapport à la composition totale.

6. Composition selon l'une quelconque des revendications précédentes, **caractérisée en ce qu'**elle a un pH dans la plage de 6,5 à 10,5.

7. Procédé d'ondulation permanente des cheveux **caractérisé en ce que** les cheveux sont lavés ou shampouinés dans un premier temps, et enroulés sur des bigoudis et qu'ensuite une composition comprenant au moins un agent de réduction, au moins un composé avec au moins deux groupes amides (composé diamide) dans sa molécule, qui est choisi parmi des composés selon la structure générale 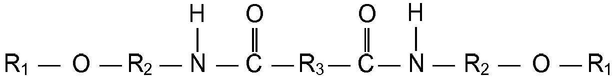 où R₁ est une chaîne alkyle linéaire ou ramifiée, saturée ou non saturée, avec de 1 à 12 atomes C qui peuvent être substitués avec des groupes hydroxyle et/ou alcoxy, R₂ est une chaîne alkyle linéaire ou ramifiée avec de 1 à 5 atomes C, et R₃ est une chaîne alkyle linéaire ou ramifiée, saturée ou non saturée, avec de 1 to 22 atomes C.et au moins un polymère cationique est appliquée sur les cheveux et à la fin d'un temps de traitement de 1 à 45 min, de préférence de 1 à 30 min, en fonction de la résistance des cheveux, est enlevé des cheveux par rinçage avec de l'eau du robinet et une composition oxydante comprenant au moins un agent d'oxydation, de préférence, du peroxyde d'hydrogène ou du bromate de sodium, à une concentration de 0,5 à 10 % en poids, calculée par rapport au total de la composition oxydante, est appliquée sur les cheveux et est laissée sur les cheveux de 1 à 20 min, et est enlevée par rinçage, et les bigoudis sont enlevés des cheveux.

8. Procédé selon la revendication 7, **caractérisé en ce que** l'agent de réduction est choisi parmi l'acide thioglycolique, l'acide thiolactique et leurs sels, la cystéine et son sel chlorhydrate, l'acétylcystéine.

9. Procédé selon les revendications 7 et 8, **caractérisé en ce qu'**au moins un composé diamide est choisi parmi les composés

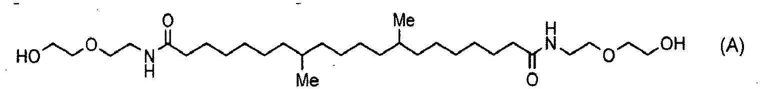

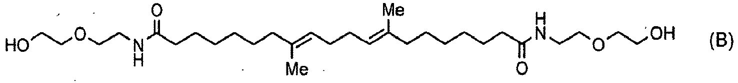

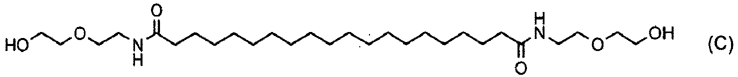

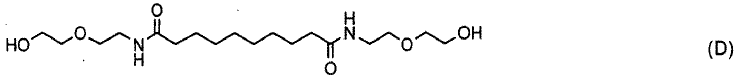

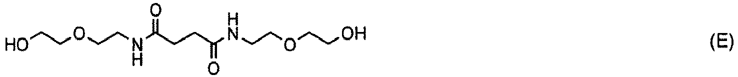

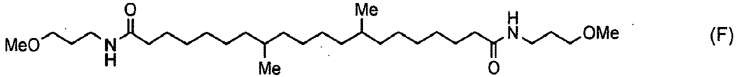

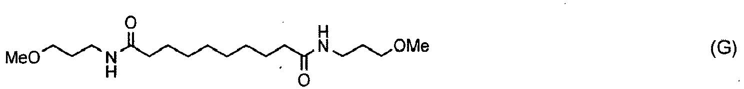

10. Procédé selon la revendication 9, **caractérisée en ce qu'**au moins un composé diamide est le

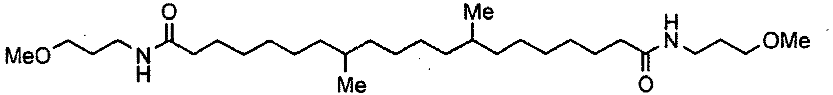

11. Procédé selon les revendications 7 à 10, **caractérisé en ce que** la composition comprend un agent de réduction à une concentration de 0,5 à 15 % en poids, un composé diamide à une concentration de 0,001 à 5 %en poids, et un polymère cationique à une concentration de 0,01 à 5 % en poids, calculées par rapport à la composition totale.

12. Procédé selon les revendications 7 à 11, **caractérisé en ce que** la composition comprend au moins un tensioactif choisi parmi les tensioactifs anioniques, non ioniques, cationiques et amphotères à une concentration de 0,05 à 10 % en poids, calculée par rapport à la composition totale.

13. Procédé selon les revendications 7 à 12, **caractérisé en ce que** la composition comprend au moins un solvant organique à une concentration de 0,1 à 10 % en poids, calculée par rapport à la composition totale.

14. Procédé selon les revendications 7 à 13, **caractérisé en ce que** la composition comprend au moins une ubiquinone selon la formule 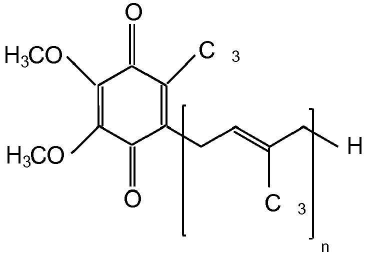 où n est un nombre entre 1 et 10.

15. Procédé selon les revendications 7 à 14, **caractérisé en ce que** la composition a un pH dans la plage de 6,5 à 10,5.
